# EUROPEAN PATENT APPLICATION

(11) **EP 4 745 977 A1**
(43) Date of publication of application: **20.05.2026**
(21) Application number: 24212726.4
(22) Date of filing: 13.11.2024
(51) Int. Cl.: G16H 10/40, G01N 35/00

(54) **A METHOD OF ASSIGNING A SET OF TESTS TO A SAMPLE CONTAINER**

(71) Applicant: Beckman Coulter, Inc., Brea, CA 92821 (US)
(72) Inventor: NAETZER, Beate, 85604 Zorneding (DE)
(74) Representative: Paustian & Partner Patentanwälte mbB

(57) **Abstract**

The present invention relates to a method, comprising:
obtaining, by a computing device, a plurality of tests to be performed by an Automated Laboratory System (ALS) on a biological sample,
determining, by the computing device among the plurality of tests, one or more tests that are to be carried out on a centrifuged portion of the biological sample and one or more tests that are to be carried on a uncentrifuged portion of the biological sample, and
for each sample container of the plurality of sample containers, assigning, by the computing device, a respective set of tests of the plurality of tests to said each sample container, such that said respective set of tests assigned to said each sample container comprises either only tests that are to be carried out on a centrifuged portion of the biological sample or only tests that are to be carried out on an uncentrifuged portion of the biological sample. In particular each sample container of the plurality of container contains or is to contain a respective portion of the biological sample.

## Description

The present disclosure relates to a method of assigning a set of tests to a sample container.

Typically, Automated Laboratory Systems (ALSs) are assemblies comprising a plurality of components which allow for processing (e.g., testing) biological samples contained in respective sample containers. Sample containers are typically used for holding samples to be tested, e.g., biological samples (for instance blood samples) drawn from a human or animal subject. An opening of the sample container may be closable, e.g. sealable, by a cap.

The components may for example include at least one of pre-processing components (e.g., an input region for inserting sample containers in the ALS, a decapper, a centrifuge, an aliquoter), a processing portion (e.g. for analysing a biological sample contained in the container) and a post-processing component (e.g., a storage unit for storing sample containers). Examples of transportation components comprise a track, a belt, and a carrier.

After introducing a sample container into an ALS, e.g. via a pre-processing component of the ALS, the sample container may be uncapped by a decapper. An analytic instrument of a processing component may be configured to carry out one or more clinical tests on the sample. An analytical instrument may be a laboratory analyzer. After analyzing the sample contained in the sample container, the sample container may be recapped by a recapper and stored in a storage device.

Some clinical tests may require centrifuged samples. For example, centrifugation is used to separate plasma samples from the blood cells (red blood cells, white blood cells and platelets), as the presence of those cells can spoil an analysis of a plasma sample.

A first aspect of the present disclosure refers to a method, comprising:
obtaining, by a computing device, a plurality of tests to be performed by an Automated Laboratory System (ALS) on a biological sample, determining, by the computing device among the plurality of tests, one or more tests that are to be carried out on a centrifuged portion of the biological sample and one or more tests that are to be carried on a uncentrifuged portion of the biological sample; and for each sample container of the plurality of sample containers, assigning, by the computing device, a respective set of tests of the plurality of tests to said each sample container, such that said respective set of tests assigned to said each sample container comprises either only tests that are to be carried out on a centrifuged portion of the biological sample or only tests that are to be carried out on an uncentrifuged portion of the biological sample. In particular each sample container of the plurality of container contains or is to contain a respective portion of the biological sample.

By providing such a method, each sample container is assigned only with one or several tests (i.e. a respective set of tests) which comprises either only tests that are to be carried out on a centrifuged portion of the biological sample or only tests that are to be carried out on an uncentrifuged portion of the biological sample. The centrifugation state of the portion contained in said each sample container (i.e. centrifuged / to be centrifuged or uncentrifuged / not to be centrifuged) desirably corresponds to the requirements of the set of tests (i.e. the requirement to be carried out on a centrifuged portion or the requirement to be carried out on an uncentrifuged portion).

Accordingly, the method of the present disclosure may avoid that a sample contained is assigned with tests which require both an uncentrifuged portion and a centrifuged portion. Consequently, an exemplary scenario can be avoided, where a sample container is centrifuged between two tests assigned to the sample container (i.e. the prior test requiring an uncentrifuged portion and the subsequent test a centrifuged portion).

Advantageously, the method of the present disclosure provides significant advantages for managing tests on biological samples in an automated laboratory system, ALS, specifically by optimizing the use of sample containers based on the centrifugation requirements. By assigning tests that require either a centrifuged or uncentrifuged portion to distinct sample containers, the method prevents the inefficiencies and potential errors caused by assigning conflicting tests to a single container.

Additionally, this method reduces the likelihood of cross-contamination between tests and simplifies the logistics of handling samples within automated systems. Time efficiency is greatly improved as sample containers do not need to be revisited for different preparation steps between tests.

Obtaining the plurality of tests may comprise accessing test data, e.g., retrieving said data from a computer memory or a database, e.g. from the memory of the computing device. Retrieving the data may comprise downloading the data. Additionally, or alternatively, accessing the data may comprise receiving the data, e.g. from another computing device or a database. For instance, accessing the plurality of tests may comprise receiving the data, storing the data in the memory of the computing device and retrieving the data by accessing said memory. For example, test data comprise the test order associated with the biological sample, i.e., the list of the tests to be carried out on the biological sample, e.g., from a Laboratory Information System (LIS). The list of the tests to be carried out on the biological sample may consist of the plurality of tests. In particular, list of the tests to be carried out on the biological sample comprises the plurality of tests, that is further comprises tests that are not comprised in the plurality of tests.

Determining the one or more tests that are to be carried out on a centrifuged portion of the biological sample and the one or more tests that are to be carried on a uncentrifuged portion of the biological sample may comprise the automated process of determining one or more tests that are to be carried out on a centrifuged portion of the biological sample and one or more tests that are to be carried on a uncentrifuged portion of the biological sample. In particular, determining the one or more tests may comprise or be implemented by a set of computer readable instructions, e.g., stored in a memory of the computing device, which when executed by the computing device, cause the computing device to determine, based on the obtained plurality of tests, one or more tests that are to be carried out on a centrifuged portion of the biological sample and one or more tests that are to be carried on a uncentrifuged portion of the biological sample. For example, the tests may be classified into two respective classes, e.g. "centrifuged portion required" and "uncentrifuged portion required". The tests may be annotated respectively. For instance, each test may be associated with respective information indicative of whether said test is to be carried out on a centrifuged portion biological samples or if said test is to be carried out on a uncentrifuged portion of the biological sample. For example, said respective information may be comprised in a respective tag associated with said test. In particular, the test data may comprise, for each test of the plurality of tests to be performed by an Automated Laboratory System (ALS), the respective information, e.g., the respective tag described above. Alternatively or additionally, the test data may comprise, for each test of the plurality of tests to be performed by an Automated Laboratory System (ALS), respective information (e.g., a link) indicative of where the respective information is stored in a computer memory and/or in a database. It is also possible that the obtained plurality of tests are already annotated with the respective two classes, e.g. "requires uncentrifuged portion" and "requires centrifuged portion". In this case, the computing device may for example reads the annotations of the respective tests, in order to determine the one or more tests that are to be carried out on a centrifuged portion of the biological sample and the one or more tests that are to be carried on a uncentrifuged portion of the biological sample.

Assigning a respective set of tests to the each sample container may comprise the automated process of assigning a respective set of tests of the plurality of tests to said each sample container. In particular, assigning a respective set of tests may comprise or be implemented by a set of computer readable instructions. In particular, this set of computer readable instructions may be stored in a memory of the computing device and when executed by the computing device, cause the computing device to assign, for each sample container of the plurality of sample containers, a respective set of tests of the plurality of tests to said each sample container, such that said respective set of tests assigned to said each sample container comprises either only tests that are to be carried out on a centrifuged portion of the biological sample or only tests that are to be carried out on an uncentrifuged portion of the biological sample, wherein each sample container of the plurality of container contains or is to contain a respective portion of the biological sample.

A portion of the biological sample may be contained or to be contained in a sample container that is not part of the plurality of sample containers described herein.

In particular, assigning a set of tests of the plurality of tests to a sample container may comprise identifying and allocating said set of tests to said sample container. In particular, assigning set of tests of the plurality of tests to a sample container may be based on one or more criteria, wherein the one or more criteria comprises the criterion that: (i) a test that is that to be carried out on a uncentrifuged portion of the biological sample (hereinafter also referred to as: "uncentrifuged test") is assigned to a sample container that is uncentrifuged and not to be centrifuged and (ii) a test that is that to be carried out on a centrifuged portion of the biological sample (hereinafter also referred to as: "centrifuged test") is assigned to a sample container that is centrifuged or is to be centrifuged. In particular, the assignment involves a controlled and rule-based process managed by a computing device to allocate tests to sample containers based on the specific characteristics of the sample portions (e.g., based on whether the sample is centrifuged or to be centrifuge or whether the sample is uncentrifuged and not to be centrifuged). This ensures that the right tests are performed on the correct type of sample, optimizing the accuracy of laboratory diagnostics.

In particular, the sets of tests may be mutually disjoint from one another. For instance, for each pair of sets of tests, the sets of said pair are disjoint from one another, i.e., they do not share any elements. In other words, each test of the plurality of tests may occur in only one set of tests and not in several sets.

The method of the present disclosure, in particular the assignment process, may be automated, i.e., it may be performed by the computing device without human intervention.

The method of the present disclosure may be a computer-implemented method.

A set of tests may comprise one or several tests, i.e., at least one test.

According to the present disclosure, an ALS may be an assembly comprising a plurality of components. The ALS may comprise a computing device operatively connected to those components and is configured to control each component. A component of the ALS may be an analytic instrument, a pre-processing (e.g. pre-analytic) instrument, a post-processing (e.g. post-analytic) instrument, an input module, an output module, a transportation component (e.g., a track, a belt, a tube carrier and the like), configured to move a sample container. In the following, "processing component" and "instrument" may be used interchangeably.

In particular, the transportation components may allow for moving containers from one laboratory instrument to another laboratory instrument or for moving containers from one component of a laboratory instrument to another component of the same laboratory instrument or to another component of another laboratory instrument. The automation components may include one or more tracks, one or more belts and/or container carriers configured to move biological samples contained in sample containers.

An analytic instrument (hereinafter referred to also as: "laboratory instrument") may be configured to carry out at least a clinical test. An analytical instrument may be a laboratory analyzer. The analytical instrument may include one or more pre-analysis, analysis, and post-analysis components.

An analyzer may be, in particular, a laboratory instrument configured to carry out one or more analytic steps, such as measuring one or more characteristics of a biological sample, e.g., the concentration of an analyte. The laboratory analyzer may include an immunoassay analyzer, a chemistry analyzer, and identification and antibiotic susceptibility analyzer, a bacteriology analyzer, a molecular analyzer, a hematology analyzer, a urinalysis instrument, and the like.

A pre-analysis laboratory instrument may be configured to carry out one or more pre-analytic steps on a biological sample to prepare the biological sample for the analytic instrument(s). A pre-analysis laboratory instrument may include or be a centrifuge, a decapper, a recapper, an input module and/or an aliquoter for preparing further sample containers containing each a portion of the biological sample. The method of the present disclosure may in particular cause pre-analytic steps (e.g., uncapping a sample container, aliquoting a part of the biological sample comprised in the sample container, (re)capping the sample container, and/or centrifuging the sample container) to be carried out.

A post-analysis laboratory instrument may be configured to carry out one or more post-processing steps on the biological sample after the biological sample has been processed by one or more laboratory analyzers. A post-analysis laboratory instrument may include one or more of a recapper (e.g., for putting caps back on containers), a storage unit, in particular a refrigerated storage unit, and an output module.

Both pre-analysis and post-analysis laboratory instruments may be referred to as peri-analytical laboratory instruments.

A biological sample may be a sample of a bodily fluid of a human or animal subject. For example, the bodily fluid may be a physiological fluid, such as blood, saliva, urine, sweat, amniotic fluid, cerebrospinal fluid, ascites fluid, or the like. The biological sample may comprise one or more components that may potentially comprise analytes of interest for performing at least one clinical test. For instance, blood serum, blood cells and blood plasma are components of a blood sample.

Typically, the biological sample is put into a sample container. The sample container may be a sample tube. Typically, sample tubes comprise a closed tube end and an end opposite thereto. The latter end defines an opening for inserting the sample in the sample tube. The opening may be closeable, e.g. sealable, by a cap. In general, a sample container may be configured to receive and hold a biological sample. Accordingly, the sample container may comprise an opening for receiving the sample and a bottom as well as one or more walls to hold the sample, wherein the opening may be configured to be releasably closed by a closing element, e.g. a cap. In particular, the sample container may have a substantially cylindrical shape (i.e. a cylindrical wall), with the opening at one base and the bottom at the opposite base.

A plurality of tests may be associated with a biological sample and may specify clinical tests to be carried out on the biological sample. The plurality of tests associated with a biological sample may be comprised in a Laboratory Information System (LIS) together with information allowing for uniquely and unambiguously associate the tests with the corresponding biological sample. The LIS may be comprised by the ALS and/or by the computing device, may be external to the ALS and/or the computing device, and/or may be associated with the ALS and/or the computing device. For example, a LIS may be associated with a plurality of ALS. The LIS and the ALS may be communicatively connected with each other, e.g. for transmitting a test order from the LIS to the ALS.

In particular, obtaining the plurality of tests may comprise obtaining, by the computing device, the test order. Obtaining the test order may comprise accessing the test order, e.g., retrieving the test order from the LIS. Retrieving the test order may comprise downloading the test order. Additionally, or alternatively, accessing the test order may comprise receiving the test order, e.g. from the LIS. The two options are not mutually exclusive. For instance, accessing the test order may comprise receiving the test order from the LIS, storing the test order in the memory of the computer device and retrieving the test order by accessing said memory.

A clinical test may comprise one or more procedures that, when carried out on the biological sample or on a component thereof, allow for estimating the value of a parameter, e.g. a clinical parameter. In particular, a clinical test may comprise physical, biological, optical, mechanical, immunological, and/or chemical procedures.

Exemplarily, an automated laboratory system processes a biological sample included in a sample container by causing the sample container to travel within the automated laboratory system by using the transportation means of the automated laboratory system so that a subset of the instruments of the automated laboratory system can carry out the pre-analytical, analytical, and post-processing steps needed to perform the clinical tests in the test order associated with the biological sample.

Each sample container contains or is to contain a respective portion of the biological sample. For instance, for at least a sample container of the plurality of sample containers the assigning of the respective set of tests may be carried out before the sample container is prepared e.g., before a portion of the biological sample is introduced in the sample. In this case, in particular, the sample container will be prepared after the assignment of the set of tests and, hence, the sample container is to contain the respective portion of the biological sample. For example, for at least a sample container of the plurality of sample containers the assigning of the respective set of tests may be carried out after the sample container is prepared. In this case, in particular, the sample container contains the respective portion of the biological sample.

In general, the method of the present disclosure may be carried out for a plurality of different biological samples.

For each sample container of the plurality of sample containers, assigning the respective set of tests of the plurality of tests to said each sample container may be such that:
- if the respective portion of the biological sample contained in said each sample container is centrifuged or is to be centrifuged, the respective set of tests comprises only tests that are to be carried out on a centrifuged portion of the biological sample, and
- if the respective portion of the biological sample contained in said each sample container is uncentrifuged and is not to be centrifuged, the respective set of tests comprises only tests that are to be carried out on an uncentrifuged portion of the biological sample.

Accordingly, each sample container may be assigned a set of tests based on the state of the biological sample it holds. If the sample portion in the container is or will be centrifuged, only tests that require a centrifuged sample portion may be assigned to that container. Conversely, if the sample portion is uncentrifuged and will remain uncentrifuged (i.e., is not to be centrifuged), the assigned tests may be those that require an uncentrifuged sample portion. In particular, a portion of the biological sample is not to be centrifuged if, when the set of tests is assigned to the sample container that contains or is to contain that portion, the portion is uncentrifuged and the instructions defining how that portion (and/or the sample container containing it) should be handled do not comprise any instructions prompting the centrifugation of that portion (and/or of the sample container containing it). More particularly, for example, the portion of the biological sample, is not centrifuged and the instructions defining how that portion (and/or the sample container containing it) should be handled do not comprise any instructions prompting the centrifugation of that portion (and/or of the sample container containing it) before the completion and/or the starting of each test of the respective set of tests assigned. In particular, this does not rule out the possibility of centrifugation after the completion and/or the starting of each test of the respective set of tests assigned to the sample containers containing that portion, e.g., if additional tests requiring centrifuged samples are assigned to that portion of the biological sample (and/or to the sample container containing it) after the first round of testing.

The respective portion of the biological sample contained or to be contained in at least one second sample container of the plurality of sample containers may be respectively aliquoted or to be aliquoted from a first sample container of the plurality of sample containers.

Accordingly, the first sample container (herein also referred to as: "parent sample container"), may be introduced into the ALS. A volume of the biological sample may be aliquoted from the first sample container into a second sample container. In particular, said volume constitutes or is comprised in the respective portion of the biological sample contained in at least one second sample container. There may exist one or more second sample containers, e.g. depending on a number and type of tests which are to be carried out on the biological sample.

For instance, an input module may be configured to receive a first sample container that is inputted in the ALS. For instance, the input module may be or comprise a loading area (e.g. an inlet) of the automated laboratory system configured to receive single sample containers or sample containers stored in a transportation rack. An input module may also be or further comprise a distribution buffer, where sample containers may be positioned before being transferred on a transportation component, e.g., on a track or a belt, to be moved within the ALS.

For example, a pre-analysis laboratory instrument may include or be an aliquoter for preparing further second sample containers containing each a portion of the biological sample contained in the first sample container.

In general, the method of the present disclosure may be carried out for a plurality of first sample containers, i.e. for a plurality of different biological samples contained in respective first sample containers. Accordingly, the input module may be configured to receive several first sample containers that are inputted in the ALS.

The method may further comprise, for at least a second sample container of the plurality of sample containers, causing the respective portion of the biological sample to be aliquoted from the first sample container into the at least second sample container.

For example, causing the portion to be aliquoted may comprise or be implemented by a set of computer readable instructions, e.g., stored in a memory of the computing device, which when executed by the computing device, cause the computing device to initiate or cause the respective portion of the biological sample contained in the at least one second sample container to be aliquoted from the first sample container, e.g. using an aliquoter of the ALS.

In particular, causing the respective portion of the biological sample contained in the at least one second sample container to be aliquoted from the first sample container may comprise causing a volume of the biological sample to be aliquoted from the first sample container to the second sample container, said volume constituting or being comprised in the respective portion of the biological sample contained in the at least one second sample container.

A portion of the biological sample may be aliquoted from the first sample container into a sample container that is not part of the aforementioned plurality of sample containers and, hence, is not one of the one or more third sample containers.

The method may further comprise, for each sample container of the plurality of sample containers different from the first sample container, causing the respective portion of the biological sample to be aliquoted from the first sample container into said each sample container.

Accordingly, in said examples, each sample container which is different from the first sample container (i.e. the parent sample container) is a respective second sample container of the one or more second sample containers, i.e., a sample container into which a respective portion has been aliquoted from the first sample container.

Generally, causing the action to be performed may comprise or be implemented by a set of computer-readable instructions, e.g., stored in a memory of the computing device, which when executed by the computing device, cause the computing device to initiate or cause the respective action to be performed by the ALS on the sample portion or sample container, such as aliquotation, centrifugation or any other action.

The respective set of tests of the plurality of tests assigned to the first sample container may comprise only tests that are to be carried out on an uncentrifuged portion of the biological sample.

Accordingly, it is not required to centrifuge the first sample container hat implies several advantages: For example, the portion of the sample contained in the first sample container (i.e. the parent sample container) can be maintained in the uncentrifuged state. Accordingly, in case a second sample container needs to be prepared e.g., by aliquoting a respective volume of the sample included in the first sample container, this can be done any point in time, independently of whether the second sample container is required to contain a centrifuged or an uncentrifuged portion of the sample.

Moreover, the first sample container is not required to be apt for being centrifuged. Since the first sample container may typically originate from any external supplier, e.g. from an external hospital, it may have e.g. a cap which is not apt for centrifugation. The container may, for example, be sealed with a stopper that has been inserted (i.e. pushed) into the container (e.g. a tube) and slips further into the container during centrifugation due to the centrifugal force. This can make it very difficult to remove the stopper from the container, which may require manual intervention. Such problems can be prevented by centrifuging only second sample containers. A second sample container may, for example, be closed with a screw cap, which essentially retains its position during centrifugation and can therefore be reliably removed again after centrifugation in an automated manner.

The method may further comprise causing at least one third sample container of the plurality of sample containers to be centrifuged, wherein the respective set of tests of the plurality of tests assigned to the third sample container may comprise only tests that are to be carried out on a centrifuged portion of the biological sample.

In particular, the third sample container may be the at least one first container described herein. In this case, the third sample container is obtained from the parent sample container through aliquoting. In this case, the method may comprise causing the at least one second sample container to be centrifuged, wherein the respective set of tests of the plurality of tests assigned to the at least one sample container may comprise only tests that are to be carried out on a centrifuged portion of the biological sample. For instance, the third sample container may be one of the one or more second sample containers.

Alternatively, the third sample container may be the first sample container, i.e., the parent sample container. In this case, in particular, the method comprises causing the first sample container of the plurality of sample containers to be centrifuged, wherein the respective set of tests of the plurality of tests assigned to the first sample container may comprise only tests that are to be carried out on a centrifuged portion of the biological sample.

In some examples, the plurality of sample containers comprises one or more third sample containers, wherein, for each sample container of the one or more sample containers, the respective set of tests assigned to said each sample container comprises only tests that are to be carried out on a centrifuged portion of the biological sample. In this case, the method may comprise the method may further comprise, causing at each sample container of the one or more sample containers to be centrifuged.

Causing the third sample container to be centrifuged may comprise one or more of: causing the third sample container to be capped with a removable cap before being centrifuged and causing the third sample container to be uncapped after being centrifuged.

For example, the third sample container may be closed with a screw cap, which essentially retains its position during centrifugation and can therefore be reliably removed again after centrifugation in an automated manner.

The respective set of tests assigned to a fourth sample container may comprise only tests that are to be carried out on a centrifuged portion of the biological sample and the respective set of tests assigned to a fifth sample container may comprise only tests that are to be carried out on an uncentrifuged portion of the biological sample.

The fourth sample container may in particular be the third sample container and/or the first sample container. In this case, more particularly, the respective set of tests assigned to the third sample container and/or respective set of tests assigned to the first sample container comprises only tests that are to be carried out on a centrifuged portion of the biological sample. For instance, the fourth sample container may be the third sample container and/or the second sample container. In this case, in particular, the respective set of tests assigned to the third sample container and/or respective set of tests assigned to the second sample container comprises only tests that are to be carried out on a centrifuged portion of the biological sample.

The fifth sample container may in particular be the first or the first sample container. In this case, in particular, the respective set of tests assigned to the second sample container and/or respective set of tests assigned to the first sample container comprises only tests that are to be carried out on an uncentrifuged portion of the biological sample.

The method may further comprise causing at least one test of the respective set of tests assigned to a sixth sample container of the plurality of sample container to be performed on a volume of the respective portion of the biological sample, the respective portion of the biological sample being contained or to be contained in the sixth sample container.

In particular, the sixth sample container may in particular be the fourth sample container which has not been centrifuged and is assigned with a respective set of tests, or the fifth sample container which has been centrifuged and is assigned with a respective set of tests.

In an exemplary scenario, the automated laboratory system (ALS) may receive the first sample container (the parent container) that contains the biological sample and is associated with a respective sample container identifier. This container may be introduced into the ALS, e.g., by a laboratory user. For example, the second container may not be designed for centrifugation (e.g., it has a stopper that could malfunction under centrifugal forces) and, hence, no centrifugation may be performed on it. The computing device may determine, based on the one or more tests that are to be carried out on a centrifuged portion of the biological sample and the one or more tests that are to be carried on a uncentrifuged portion of the biological sample, the number, *N*, of the sample containers of the plurality of sample containers. The computing device may assign, to each sample container of the plurality of sample containers a respective set of tests, such that said respective set of tests assigned to said each sample container comprises either only tests that are to be carried out on a centrifuged portion of the biological sample or only tests that are to be carried out on an uncentrifuged portion of the biological sample. In particular, the plurality of sample containers comprises the first sample container and one or more, say *(N-1)* second sample containers that when, the tests assignation is carried out, are not prepared yet.

Next, in this exemplary scenario, the one or more second sample containers are created by aliquoting respective portions of the biological sample from the first sample container, thereby obtaining the plurality of sample containers including the first sample container and the one or more second sample containers. For example, the computing device may cause, e.g., by instructing the aliquoter of the ALS to aliquot sample portions from the parent container into the one or more second sample containers.

The plurality of sample containers may comprise one or more third sample containers, i.e., sample containers to which are assigned only tests that are to be carried out on a centrifuged portion of the biological sample. If the second container is not be designed for centrifugation, that sample container is not one of the one or more third sample container. The computing device may then instruct the centrifuge of the ALS to centrifuge the sample containers of the plurality of sample containers to which a set of centrifuged tests are assigned. This structured process provides efficient sample management and reduces errors in processing.

The method may further comprise:
obtaining information specifying that a first test is to be carried out on the biological sample, wherein said information is based on a result of a second test of the plurality of tests,
determining whether the first test is to be carried out on a centrifuged portion of the biological sample or is to be carried on a uncentrifuged portion of the biological sample; if the first test is to be carried out on a uncentrifuged portion of the biological sample, assigning the first test to a sample container or the plurality of sample container, such that the respective set of tests assigned to said sample container comprises only tests that are to be carried out on a uncentrifuged portion of the biological sample.

For instance, obtaining the information may comprise determining, based on the result of the second test, that the first test is to be carried on the sample. In this case, in particular, a rerun or a reflex test (i.e., the first test) is to be carried to confirm or dispute the result of a prior test (i.e., the second test). In those examples, the first test is assigned to sample container depending on whether the first test requires a centrifuged or an uncentrifuged portion of the biological sample. The first and second tests may be the same test, i.e., the result of the second test triggers a rerun of said test. For example, the first test may be the same test type of the second test, but it is to be carried out by a different laboratory analyzer. For instance, the first and second tests may be different from each other, i.e., the result of the second test triggers a reflex test, i.e., the first test.

The method may further comprise, if the first test is to be carried out on a centrifuged portion of the biological sample, assigning the first test to a sample container of the plurality of sample containers, such that the respective set of tests assigned to said sample container comprises only tests that are to be carried out on a centrifuged portion of the biological sample.

In those examples, the first test is assigned to a sample container that is or is to be centrifuged, which streamlines the processing of the plurality of sample containers, as the performance of the first step does not require the centrifugation of the sample container to which the first test is assigned.

The method may further comprise, if the first test is to be carried out on a centrifuged portion of the biological sample, determining whether at least one sample container among one or more sample containers comprise a volume of biological sample that is sufficient for carrying out the first test, wherein the one or more sample containers are comprised in the plurality of sample containers and such that the respective sets of tests assigned to said one or more sample containers comprise only tests that are to be carried out on a centrifuged portion of the biological sample.

In particular, in those examples, the first test is assigned to a centrifuged or a to be centrifuged sample container if one at least one of the centrifuged or a to be centrifuged sample containers contains enough volume of sample container to carry out the first test. In particular, the volume of biological sample needed to carry out comprises the volume needed by the laboratory analyzers to measure the one or more analyte that the first test is to be measure and, optionally the dead volume.

The method may further comprise, if at least one sample container among one or more sample containers comprises a volume of biological sample that is sufficient for carrying out the first test:
assigning the first test to the at least one sample container; and
if no sample container among the one or more sample containers comprises a volume of biological sample that is sufficient for carrying out the first test:
   assigning the first test to a further sample container; and
   causing a portion of the biological sample to be aliquoted from a seventh sample container of the plurality of sample containers into the further sample container, wherein the respective set of tests assigned to the seventh sample container comprises only tests that are to be carried out on an uncentrifuged portion of the biological sample. In particular, the method may further comprise causing the further sample container to be centrifuged.

In particular, in those examples, the first test is assigned to a tube container that is aliquoted from the seventh sample container. The respective set of tests assigned to the seventh sample container comprises only tests that are to be carried out on an uncentrifuged portion of the biological sample, e.g., the seventh sample container is not centrifuged. For instance, the seventh sample container may be the second sample container or the first sample container. In particular, the seventh sample container is not one of the one or more third sample containers.

Assigning a respective set of tests of the plurality of tests to a sample container and causing the respective portion of the biological sample to be aliquoted from the second sample container into the at least one first sample container may be carried out in any chronological order, i.e. the assignment may be carried out before, simultaneously or after the causing the respective portion of the biological sample to be aliquoted from the second sample container.

Assigning a respective set of tests of the plurality of tests to a sample container and causing at least one third sample container of the plurality of sample containers to be centrifuged may be carried out in any chronological order, i.e. the assignment may be carried out before, simultaneously or after causing the at least one third sample container of the plurality of sample containers to be centrifuged.

Assigning a respective set of tests of the plurality of tests to a sample container may be carried out before causing at least one test of the respective set of tests assigned to a sixth sample container of the plurality of sample container to be performed on a volume of the respective portion of the biological sample contained or to be contained in the sixth sample container.

Assigning a respective set of tests of the plurality of tests to a sample container may be carried out before causing, by the computing device, the respective set of tests to be carried out on at least a volume of the respective portion of the biological sample.

The method may further comprise, for each sample container of the plurality of containers, causing, by the computing device, the respective set of tests to be carried out on at least a volume of the respective portion of the biological sample.

For each sample container of the plurality of containers, causing the respective set of tests to be carried out on the respective portion of the biological sample may comprise instructing the ALS, e.g., one more of the components of the ALS, to carry out the respective set of tests on the respective portion of the biological sample.

In particular, the computing device may be comprised in and/or communicatively connected with the ALS and may be send instructions to the ALS and/or to one or more components of the ALS, such that, for each sample container of the plurality of containers, the respective set of tests are carried out on the respective portion of the biological sample e.g., by one or more laboratory analyzers of the ALS.

According to the present disclosure, causing an action to be carried out may comprise instructing e.g., a component of the ALS, to carry out that actions. In particular, causing an action to be carried out may comprise initiating the action e.g., by instructing a component of the ALS, to carry out that actions. Causing an action to be carried out may comprise carrying out the action.

For example, causing a portion of the biological sample to be aliquoted from a sample container into a further sample container may comprise one or more of: instructing an aliquoter to aliquot that portion of the biological sample from the sample container into the further sample container, initiating the aliquoting of that portion from the sample container into the further sample container; and aliquoting, by an aliquoter of the ALS, that portion from the sample container into the further sample container.

Causing a sample container to be centrifuged may comprise one or more of: instructing a centrifuge of the ALS to centrifuge that sample container; initiating the centrifugation of that sample container, e.g., by instructing a centrifuge of the ALS; and centrifuging, by a centrifuge of the ALS, that sample container.

For instance, causing one or more tests to be performed may comprise one or more of: instructing the ALS to perform the one or more tests; initiating the performance of the one or more tests, e.g., by instructing the ALS; and performing by the ALS the one or more tests.

A second aspect of the present disclosure relates to a computing device (i.e. a data processing system) comprising a processor configured to carry out the method according to the first aspect of the present disclosure. For example, the computing device may further comprise at least one memory storing computer-executable instructions which, when executed by the processor cause the computing device and/or its processor to carry out the method according to the present disclosure.

The processor (or processing unit) may be a component of electronic devices that may be responsible for carrying out computational tasks. There may be different types of processing units, each designed for specific purposes. A processing unit may be or may comprise a Central Processing Unit (CPU), Graphics Processing Unit (GPU), Digital Signal Processor (DSP), Field-Programmable Gate Array (FPGA), and/or Application-Specific Integrated Circuit (ASIC).

A third aspect of the present disclosure relates to an automated laboratory system, ALS, comprising the computing device according to the second aspect of the present disclosure, as described above, and/or configured to carry out the method according to the present disclosure.

A fourth aspect of the present disclosure relates to a computer program product comprising instructions which, when the program is executed by a computer system, cause the computer system to carry out the method according to the present disclosure.

The computer system may be the computing device or a part of the computing device according to the second aspect of the present disclosure.

A fifth aspect of the present disclosure relates to a computer-readable medium, e.g., a non-transitory computer-readable medium, comprising instructions which, when executed by a computer system, cause the computer system to carry out the method according to the first aspect of the present disclosure.

In case the method comprises any steps (for example physical) which go beyond a mere data processing operation (for example processing the sample container), the computer program or computer-readable medium may further comprise computer-readable instructions which when executed by a data processing system or by computing device cause the ALS (or any elements or components of the ALS) to carry out these steps.

It is intended that combinations of the above-described elements and those within the specification may be made, except where otherwise contradictory.

It is to be understood that both the foregoing general description and the following detailed description are exemplary and explanatory only, are provided for illustration purposes and are not restrictive for the scope as defined by the accompanied claims.

The accompanying drawings, which are incorporated in and constitute a part of this specification, illustrate examples of the disclosure and together with the description, and serve to support and illustrate the principles thereof.
Fig. 1 shows a schematic representation of a computing device and a schematic representation of an automated laboratory system.
Fig. 2a shows a flowchart of an exemplary method.
Fig. 2b shows a flowchart of a further exemplary method.
Fig. 3a show a first exemplary flowchart of step 450 of fig. 2b.
Fig. 3b show a second exemplary flowchart of step 450 of fig. 2b.
Fig. 4 shows an exemplary flowchart of step 430 of fig. 2a and fig. 3a.
Fig. 5 shows a flowchart 500 of an exemplary method which may be carried out after the method of fig. 2a or the method of fig. 2b.

In the following text, a detailed description of examples will be given with reference to the drawings. Various modifications to the examples may be made. In particular, one or more elements of one example may be combined and used in other examples to form new examples.

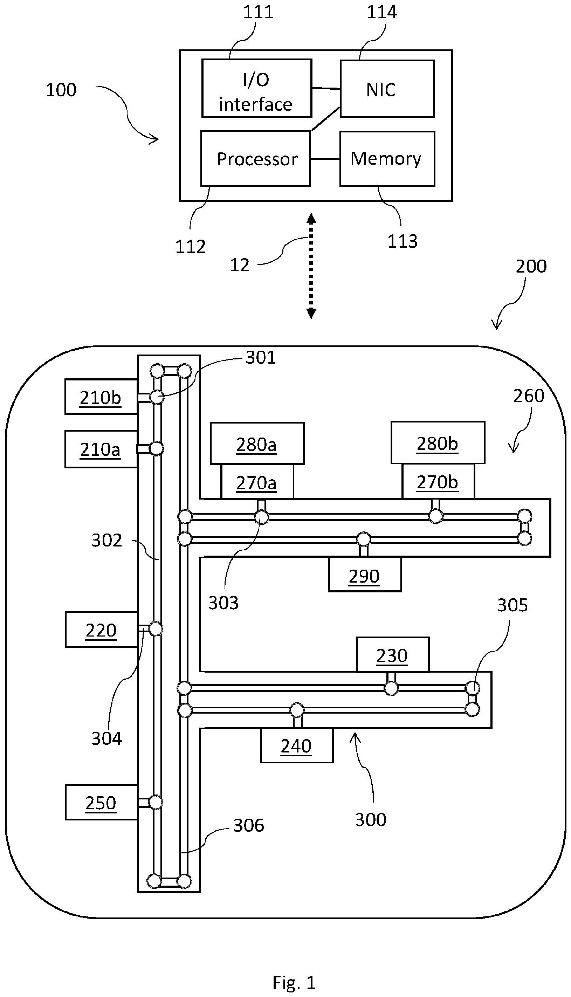

**Fig. 1** shows a schematic representation of a computing device and a schematic representation of an automated laboratory system, ALS, that may be used in some examples of the present disclosure.

The computing device 100 and the automated laboratory system (ALS) 200 may be part of a laboratory. The computing device 100 may include an input/output (I/O) interface 111, a processor 112, a memory 113, and a network interface controller 114. One or more peripheral (input or output) devices (not shown) may be connectable to the input/output interface 111. The peripheral input devices may include one or more of the following: a computer keyboard and/or a pointing device, such as a mouse. The peripheral output devices may include one or more of the following: a terminal, a printer, a disk drive or other storage device, and a video monitor. The processor 112 may include a central processing unit (CPU) and/or a graphics processing unit (GPU), each having one or more processing cores. The memory 113 may include primary memory, such as random access memory (RAM), read-only memory (ROM) or flash memory, and/or secondary memory, such as a solid-state drive or a hard disk drive.

The network interface controller 114 may be connectable to a computer network 12. The ALS 200 may also be connectable to the computer network 12. In some cases, the computing device 100 may be included in (i.e., may be part of) the ALS 200.

The ALS 200 may include a plurality of components. For instance, the ALS 200 comprises a plurality of pre-processing instruments 210a, 210b, 220. In particular, the ALS comprises one or a plurality, e.g., two, input modules 210a, 210b. Each of the input modules 210a 210b may comprise a respective decapper for decapping sample containers (not shown) and/or at least a respective centrifuge for centrifuging sample containers (not shown). In particular, the centrifuge may be configured to centrifuge a sample container, e.g., when receiving a respective instruction from the computing device 100. Alternatively, each input module of the plurality of input modules 210a, 210b may be associated with a respective centrifuge (not shown) which is mechanically connected with that input module such that sample containers may be transferred from that input module and the respective centrifuge and *vice versa.* The ALS 200 may comprise an aliquoter 220. The aliquoter is configured to aliquote a portion of a biological sample contained in a first sample container into one or several second sample containers. In particular, the aliquoter 220 may be configured to carry out the aliquoting, when receiving a respective instruction from the computing device 100. In some cases, at least an input module of the plurality of modules 210a, 210b may comprise two or more centrifuges. Exemplarily, at least one input module of the plurality of modules 210a, 210b does not comprise any centrifuge.

Each input module of the plurality of the input modules 210a, 210b may comprise a respective loading area (not shown) for loading sample containers (not shown) in the ALS 200 and for identifying said containers. Moreover, each input module of the plurality of input modules 210a, 210b may comprise a respective distribution buffer (not shown), where sample containers may be positioned before being transferred on a transportation component, e.g., on a track or a belt, to be moved within the ALS 200 for processing.

The ALS 200 comprises one or more processing instruments, i.e., analyzers, 230, 240, 250 for performing tests with a biological sample contained in sample containers (not shown). For example, the ALS 200 comprises a clinical chemistry analyzer 230, an immunoassay analyzer 240 and a hematology analyzer 250. The ALS 200 may also include more or less of these components. For instance, the ALS 200 may comprise a plurality of clinical chemistry analyzers, a plurality of immunoassay analyzers, and/or a plurality of hematology analyzers. The ALS 200 may comprise further analyzers, e.g., one or more urinalysis analyzers (not shown) and/or one or more microbiology analyzers (not shown). The ALS 200 may include one or more identification and antibiotic susceptibility analyzers, one or more bacteriology analyzers, and/or one or more molecular analyzers.

The ALS 200 comprises a plurality of post-processing instruments 270a, 270b, 280a, 280b, 290. In particular, the ALS may comprise a plurality of, e.g., two, recappers 270a, 270b for recapping sample containers and a plurality of, e.g., two, storage units 280a, 280b for storing tube containers. In particular, each recapper 270a, 270b is associated with a respective storage unit 280a, 280b. For example, the recapper 270a and the recapper 270b are associated with the storage unit 280a and the storage unit 280b, respectively. In particular, a recapper associated with a storage unit is mechanically connected with that storage unit such that sample containers may be transferred from that input module and the respective centrifuge and *vice versa.*

Alternatively, a storage unit of the ALS 200 may comprise a respective recapper associated therewith. A storage unit of the ALS 200 may further comprise a respective storage space (not shown) for storing a plurality of sample containers. The storage space may have several slots (e.g. one slot for one sample container). For example, a sample container may be recapped and then placed in a storage space (i.e., a particular slot) of a storage unit.

The above-mentioned instruments 210a, 210b, 220, 230, 240, 250, 270a, 270b, 280a, 280b, 290 may be (at least indirectly) mechanically connected with each other via a transportation system 300 such that a sample container may be transferred from any of the above-mentioned instruments 210a, 210b, 220, 230, 240, 250, 270a, 270b, 280a, 280b, 290 to any other instrument 210a, 210b, 220, 230, 240, 250, 270a, 270b, 280a, 280b, 290 by means of the transportation system 300.

Specifically, the transportation system 300 includes a plurality of transport components 301, 302. In particular, the transport system may comprise a plurality of diverters 301, 303, 305 and a plurality of tracks and/or belts, 302, 304, 306. A diverter 301, 303, 305 is in particular a mechanical device which allows to transfer a sample container from a track and/or belt to another track and/or belt. In particular, a diverter, depending on the path that a sample container shall follow, allow for changing the direction of motion of said sample container. In Fig. 1, diverters are schematically depicted by white circles 301, 303, 305

The transportation system 300 comprises a plurality of tracks and/or belts 302, 304, 306 for transporting sample containers e.g., in container carriers (not shown). In Fig. 1 tracks and/or belts are schematically depicted by white rectangles 302, 306 comprised between two diverters and white rectangles 304 comprised between a diverter and an instrument. In particular, the transport components are configured to move sample containers within the ALS 200. The transport components are only schematically indicated in fig. 1. The transportation system 300 may comprise more or less transport components. For example, the transportation system 300 may comprise one or more robotic grippers (not shown) and/or one or more container carriers (not shown).

The transportation system 300 may further comprise at least one buffer region (not shown) configured to temporarily store one or more of sample containers. The buffer region may be mechanically connected with any one of the instruments 210a, 210b, 220, 230, 240, 250, 270a, 270b, 280a, 280b, 290 of the ALS 200 such that a sample container may be transferred from any of the above-mentioned instruments to the buffer region by means of the transport components of the transportation system 300.

A sample container may thus be transferred along a determined path from an input module 210a, 210b to one of the storage units 280a, 280b, in particular along a determined path. In particular, a path comprises one or more of the instruments 220, 230, 240, 250, and a plurality of the transportation components. The plurality of transportation components allows the sample container to travel in the instruments comprised in said path so that said instruments may pre-process, post-process and/or process the biological sample contained in the sample container. Alternatively, or additionally, the path may comprise a plurality of transportation components that allows the sample container to travel in the vicinity of one or more instruments that are not comprised in the route, so that said instruments are able to retrieve and at least a portion of the biological sample contained in the sample container, and pre-process, process and/or post process said portion.

In some examples, the computing device may be comprised, e.g., part of the ALS

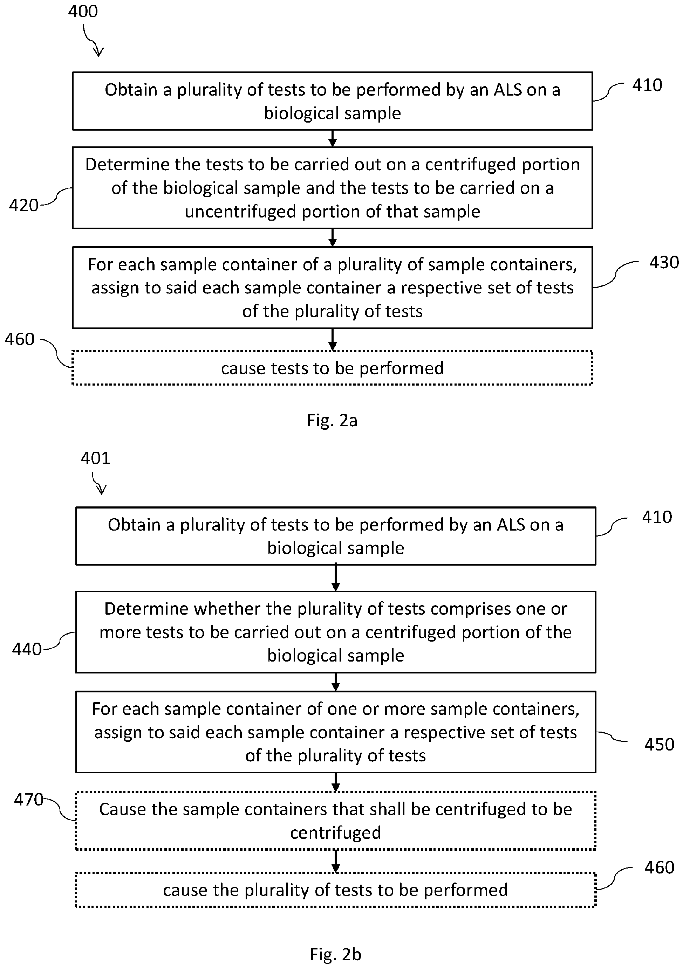

**Fig. 2a** shows a flowchart 400 of an exemplary method.

The method may be carried out by a computing device, e.g., it may be a computer implemented method, and in particular by the computing device 100 schematically depicted in fig. 1 and described above.

Step 410 of the method comprises obtaining, by the computing device, a plurality of tests to be performed by an ALS 200 on a biological sample. The biological sample may be associated with a subject, in particular a human (e.g. a patient) or an animal subject. A biological sample may be a sample of a bodily fluid of a human or animal subject. For example, the bodily fluid may be a physiological fluid, such as blood, saliva, urine, sweat, amniotic fluid, cerebrospinal fluid, ascites fluid, or the like. The biological sample may comprise one or more components that may potentially comprise analytes of interest for performing at least one clinical test. For instance, blood serum, blood cells and blood plasma are components of a blood sample.

The computing device may receive the plurality of tests from a Laboratory Information System (LIS), optionally together with information allowing for uniquely and unambiguously associate the tests with the corresponding biological sample. The LIS (not shown) may be external to the ALS 200 and/or to the computing device 100. The LIS may serve e.g. as a Hospital Information System (HIS) and/or may allow a user, e.g. a doctor, to input one or more tests related to a subject, said tests to be performed on the biological sample of said subject. The LIS and the ALS 200may be communicatively connected with each other, e.g. for transmitting a test order from the LIS to the ALS 200. Obtaining the plurality of tests may comprise accessing the plurality of tests, e.g., retrieving the plurality of tests from the LIS. Retrieving the plurality of tests may comprise downloading the plurality of tests. Additionally, or alternatively, accessing the plurality of tests may comprise receiving the plurality of tests, e.g. from the LIS. The two options are not mutually exclusive. For instance, accessing the plurality of tests may comprise receiving the plurality of tests from the LIS, storing the plurality of tests in the memory of the computer device and retrieving the plurality of tests by accessing said memory.

Each of the plurality of tests may be associated with the biological sample. For example, the biological sample may be contained in at least one sample container (as explained in more detail below) comprising a unique identifier. Thus, by associating the tests with the identifier, the tests can also be unambiguously associated with the biological sample.

Step 410 may also comprise obtaining, for each of a plurality of biological samples, each biological sample being associated with a respective subject of a plurality of subjects, a respective plurality of tests to be performed by the ALS 200 on said each biological sample.

At step 420, the computing device 100 determines the tests which are to be carried out on a centrifuged portion of the biological sample and the tests which are to be carried on a uncentrifuged portion of that sample.

For example, some clinical tests may require centrifuged samples. Centrifugation may e.g. be used to separate plasma samples from blood cells (red blood cells, white blood cells and platelets), as the presence of those cells can spoil an analysis of a plasma sample.

Step 420 may comprise an automated process of determining one or more tests that are to be carried out on a centrifuged portion of the biological sample and one or more tests that are to be carried on a uncentrifuged portion of the biological sample. In particular, determining the one or more tests may comprise or be implemented by a set of computer readable instructions, e.g., stored in a memory of the computing device, which when executed by the computing device, cause the computing device to determine, based on the obtained plurality of tests, one or more tests that are to be carried out on a centrifuged portion of the biological sample and one or more tests that are to be carried on a uncentrifuged portion of the biological sample. For example, the tests may be classified into two respective classes, e.g. "centrifuged portion required" and "uncentrifuged portion required". The tests may be annotated respectively. For instance, each test may be associated with respective information indicative of whether said test is to be carried out on a centrifuged portion biological samples or if said test is to be carried out on a uncentrifuged portion of the biological sample.

At step 430, for each sample container of a plurality of sample containers, a respective set of tests of the plurality of tests is assigned to said each sample container. Accordingly, each sample may be assigned with a respective, i.e. a distinct, set of tests. This assignment is done such that said respective set of tests assigned to said each sample container comprises either only tests that are to be carried out on a centrifuged portion of the biological sample or only tests that are to be carried out on an uncentrifuged portion of the biological sample wherein each sample container of the plurality of container contains or is to contain a respective portion of the biological sample.

For each sample container of the plurality of sample containers, assigning the respective set of tests of the plurality of tests to said each sample container may be such that, if the respective portion of the biological sample contained in said each sample container is centrifuged or is to be centrifuged, the respective set of tests comprises only tests that are to be carried out on a centrifuged portion of the biological sample, and if the respective portion of the biological sample contained in said each sample container is uncentrifuged and is not to be centrifuged, the respective set of tests comprises only tests that are to be carried out on an uncentrifuged portion of the biological sample.

The plurality of sample containers may comprise a first sample container and one or several second sample containers. The first sample container may also be referred to as a parent sample container which e.g. is introduced in an input module, say the input module 210a, of the ALS 200 e.g., by a laboratory technician. The at least one second sample container may comprise a sample portion which is aliquoted from the first sample container. Moreover, at least one of the sample containers may be centrifuged. In particular, one or several second sample containers may be centrifuged.

The first sample container may be identified upon receipt, by a barcode reader (not shown) of the input module which reads identification data comprised in a barcode attached to the sample container. In particular, the identification data comprise information that uniquely identifies the sample container and allows the computing device 100 to obtain, e.g., by querying the LIS (not shown), the plurality of tests associated with the biological sample contained in the first sample container. In particular, the ALS 200 provides the identification data to the computing device 100, which identifies the sample container by using said data.

The identification data may comprise a main identifier and a suffix. The main identifier may identify the biological sample. Accordingly, in case one or more second sample containers exist or are to be prepared, they contain the same main identifier.

Step 430 may comprise grouping the plurality of tests into several sets of tests, e.g. a first set comprising all tests of the plurality of tests requiring a centrifuged sample portion, and a second set comprising all tests of the plurality of tests requiring an uncentrifuged sample portion. However, it is also possible that the plurality of tests may be grouped into more than two sets of tests. For example, the tests of the plurality of tests requiring a centrifuged sample portion may be grouped into several mutually disjoints sets of tests (i.e. such that each test is comprised in only one set), and/or the tests of the plurality of tests requiring an uncentrifuged sample portion may be grouped into several, mutually disjoints sets of tests. In particular, step 430 may be carried out before, simultaneously or after receiving the first sample container by the ALS and/or aliquoting.

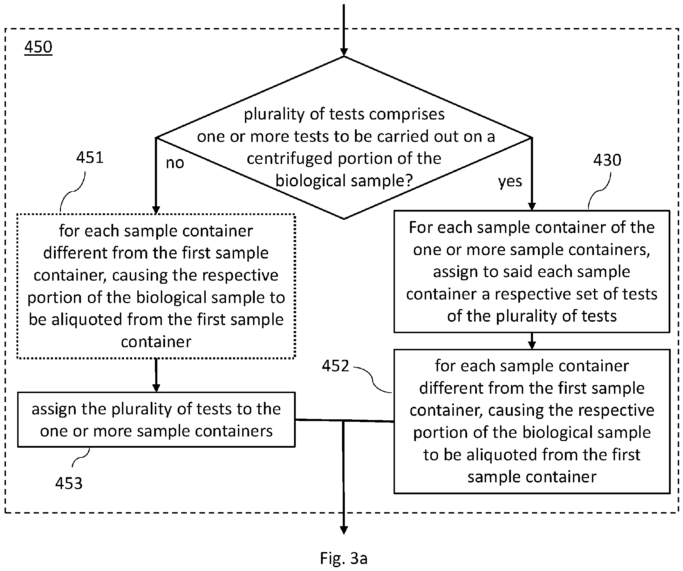

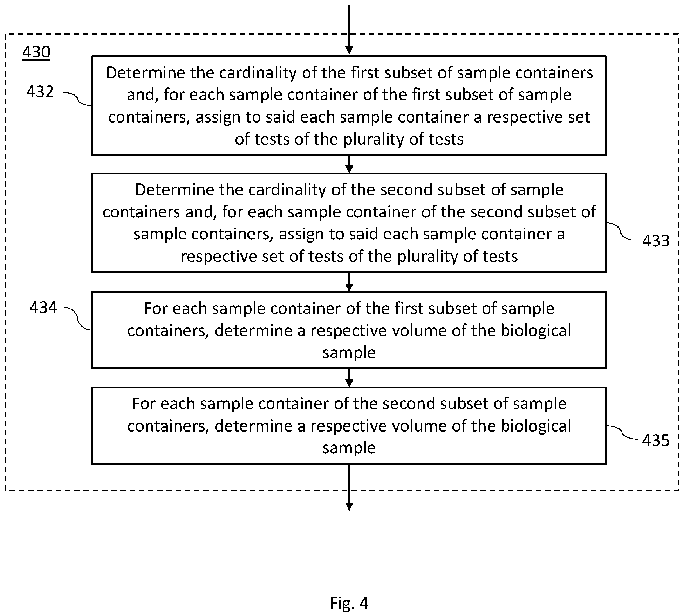

**Fig. 4** show an exemplary flowchart of step 430, as schematically depicted in fig. 2a and fig. 3a.

At step 432, the computing device 100 determines the cardinality of a first subset of sample containers and, for each sample container of the first subset of sample containers, assigns to said each sample container a respective set of tests of the plurality of tests. In particular, the first subset of sample containers consists of the sample containers of the plurality of sample containers to which is assigned a respective set of tests which consists of centrifuged tests. The cardinality of a set or a subset is the number of elements of that set or subset. Hence, in particular, the cardinality of the first subset of sample containers is equal to the number of sample containers of the plurality of sample containers to which is assigned a respective set of tests which consists of centrifuged tests.

In some examples, the determination and the assignment carried out at step 432 is based on the centrifuged tests comprised in the plurality of tests and a set of rules. The set of rules may be implemented by computer readable instructions e.g., stored in the memory of the first computing device 100. In particular, the set of rules may be specified by a lab technician. For instance, one or more rules of the set of rules may specify under which conditions an aliquot of a biological sample shall be prepared and a test shall be assigned to that portion, which is dedicated exclusively to the performance of said test. For example, such rules may read as follows:
If (test T1 is comprised in the test order of biological sample) then (create an aliquot of the biological sample and assign only test T1 to that aliquot). (1)

Such rules may be defined because the test is a high sensitivity test which provides reliable results only if performed on an uncontaminated biological sample, and/or because the test is to be carried out on a standalone instrument and hence shall be taken out of the ALS 200, and/or because the test takes long time and should be parallelized with other tests.

In these cases, the determination and the assignment of step 432 shall be carried out so that the requirements defined by the set of rules are fulfilled. For example, if the plurality of tests comprises three centrifuged tests, T1, T2 and T3, and if the set of rules consists of the rule (1), the number of sample containers in the first subset is be equal to two. Moreover, test T1 may be assigned to one sample container of the first subset and tests T2 and T3 may be assigned to the other sample container.

Exemplarily, if the set of rules consists of rule (1) and rule (2) below:
If (test T2 is comprised in the test order of biological sample) then (create an aliquot of the biological sample and assign only test T2 to that aliquot), (2)
the number of sample containers in the first subset is equal to three and the first subset consists of sample container C1, C2 and C3. Moreover, test T1 may be assigned to C1, test T2 may be assigned to C2 and test T3 may be assigned to C3. For example, one of the three tests T1, T2, T3 may be the NT-proBNP assay, which is an immunology test that can be carried out by the immunoassay analyzer 240.

In some cases, the determination and the assignment carried out at step 432 is based on the centrifuged tests comprised in the plurality of tests and an aliquoting algorithm aiming at maximizing throughput and/or minimizing turnaround time of the ALS 200. In particular, the aliquoting algorithm may be configured to receive a set of tests as inputs. The aliquoting algorithm may be configured to process the input to provide the number of sample containers that allows for maximizing throughput and/or minimizing turnaround and the tests of the set of tests assigned to each tube. In these cases, the determination and the assignment of step 432 is carried out by processing the centrifuged tests of the plurality of test by using the aliquoting algorithm.

At step 433, the computing device 100 determines the cardinality of a second subset of sample containers and, for each sample container of the second subset of sample containers, assigns to said each sample container a respective set of tests of the plurality of tests. In particular, the second subset of sample containers consists of the sample containers of the plurality of sample containers to which is assigned a respective set of tests which consists of uncentrifuged tests.

In some examples, the determination and the assignment carried out at step 433 is based on the uncentrifuged tests comprised in the plurality of tests and the set of rules described above. In these cases, the determination and the assignment of step 432 shall be carried out so that the requirements defined by the set of rules are fulfilled.

For instance, if the plurality of tests comprises three uncentrifuged tests, T1, T2 and T3, and if the set of rules consists of the rule (1), the number of sample containers in the first subset is equal to two. Moreover, test T1 is assigned to one sample container of the first subset and tests T2 and T3 are assigned to the other sample container. If the set of rules consists of rule (1) and rule (2) defined above, the number of sample containers in the first subset is equal to three and the first subset consists of sample container C1, C2 and C3. Moreover, test T1 is assigned to C1, test T2 is assigned to C2 and test T3 is assigned to C3. For example, one of the three tests T1, T2, T3 may be Blood Cell Count (BCC) test, which is to be performed by the hematology analyzer 250. A further test of the three tests T1, T2, T3 may be the HBA 1C test, which is a clinical chemistry test that measures the amount of glucose attached to the hemoglobin.

In some cases, the determination and the assignment carried out at step 433 may be based on the centrifuged tests comprised in the plurality of tests and the aliquoting algorithm described above. In these cases, in particular, the determination and the assignment of step 432 is carried out by processing the centrifuged tests of the plurality of test by using the aliquoting algorithm.

At step 434, for each sample container of the first subset of sample containers, a respective volume of the biological sample is determined. In particular, for each sample container of the first subset, the respective volume is determined based on the volume needed to carry out the tests of the set of tests assigned to said each sample container and the dead volume for said tests. More particularly, for each sample container of the first subset, the respective volume is the sum of the volume needed to carry out the tests of the set of tests assigned to said each sample container and the dead volume for said tests.

At step 435, for each sample container of the second subset of sample containers, a respective volume of the biological sample is determined. In particular, for each sample container of the first subset, the respective volume is determined based on the volume needed by the tests of the set of tests assigned to said each sample container and the dead volume for said tests.

The steps of fig. 4 may also have another chronological order or be carried out simultaneously. In particular, step 434 may be carried out before step 433 and after step 432. In some examples, steps 433 and 435 may be carried out before step 432 and 434..

With reference to fig. 2a, the flowchart 400 may comprise optional step 460. At optional step 460, the tests are caused to be performed. For instance, step 460 comprises instructing, by the computing device 100, the ALS 200 to perform the tests, thereby initiating the performance of said tests. For example, the computing device may provide to the ALS 200 instructions specifying that the ALS 200 shall perform the tests. In particular, step 460 comprises performing the tests, e.g., in response to the instructions provided by the computing device 100 to the ALS 200.

**Fig. 2b** shows a flowchart 401 of a further exemplary method. The further method comprises steps 410, as described above and schematically shown in fig. 2a.

At step 440, the computer device 100 determines whether the plurality of tests comprises one or more tests to be carried out on a centrifuged portion of the biological sample. If the plurality of tests comprises at least a centrifuged test and at least an uncentrifuged test, step 440 may in particular comprise step 420 as described above and schematically depicted in fig. 2a.

At step 450, for each sample container of one or more sample containers, the computing device 100 assigns to said each sample container a respective set of tests of the plurality of tests.

**Fig. 3a** show a first exemplary flowchart of step 450 schematically depicted in fig. 2b. In case it has been determined in step 440 that none of the tests is to be carried out on a centrifuged portion (i.e. all tests are to be carried out on an uncentrifuged portion), optional step 451 and step 453 may be carried out.

At optional step 451, for each second sample container different from the first sample container, a respective portion of the biological sample of the first sample container is caused to to be aliquoted into said each second sample container. In particular, step 451 may be carried out only if one or more rules of the set of rules described above are fulfilled. In particular, this step may comprise instructing, by the computing device 100, the ALS 200 to aliquot the respective portion of the biological sample of the first sample container into each of the second sample containers, thereby initiating the aliquoting. For example, the computing device 100 may provide to the ALS 200 instructions specifying that the ALS 200 shall aliquot the respective portion of the biological sample of the first sample container into each of the second sample containers. In particular, step 451 comprises aliquoting, by the aliquoter 220 of the ALS 200, a respective portion of the biological sample of the first sample container into each of the second sample containers. For example, based on the instructions received by the computing device 100, the ALS 200 may instruct the aliquoter 220 to aliquot a respective portion of the biological sample contained in the first sample container into each of the second sample containers.

Accordingly, in this case, each sample container of the one or more sample containers contains a sample portion that is uncentrifuged and not to be uncentrifuged. At step 453, the plurality of tests may be assigned to the one or more sample containers. If optional step 451 is not carried out, the tests may be assigned to a single sample container, e.g. the uncentrifuged first sample container.

In case it has been determined at step 440 that at least one test is to be carried out on a centrifuged portion, steps 430 and 452 are carried out. In particular, step 430 is identical to the homonymous step schematically depicted in figure 2a and figure 4, as described above.

At step 452, for each second sample container, i.e. for each sample container of the plurality of sample containers different from the first sample container, the respective portion of the biological sample may be caused to be aliquoted from the first sample container. In particular, this step may comprise instructing, by the computing device 100, the ALS 200 to aliquot a respective portion of the biological sample of the first sample container into each of the second sample containers, thereby initiating the aliquoting. For example, step 452 comprises aliquoting, by the aliquoter 220 of the ALS 200, a respective portion of the biological sample of the first sample container into each of the second sample containers. For example, based on the instructions received by the computing device 100, the ALS 200 may instruct the aliquoter 220 to aliquot a respective portion of the biological sample contained in the first sample container into each of the second sample containers.

In particular, in some examples, for each sample container of the plurality of sample containers which is different from the first sample container and is comprised in the first subset of sample containers described above, the volume of the respective portion of sample container is equal to the respective volume of the biological sample, as determined at step 434 of fig. 4. For instance, in some examples, for each sample container of the plurality of sample containers which is different from the first sample container and is comprised in the second subset of sample containers described above, the volume of the respective portion of sample container is equal to the respective volume of the biological sample, as determined at step 435, as described above (cf. fig. 4).

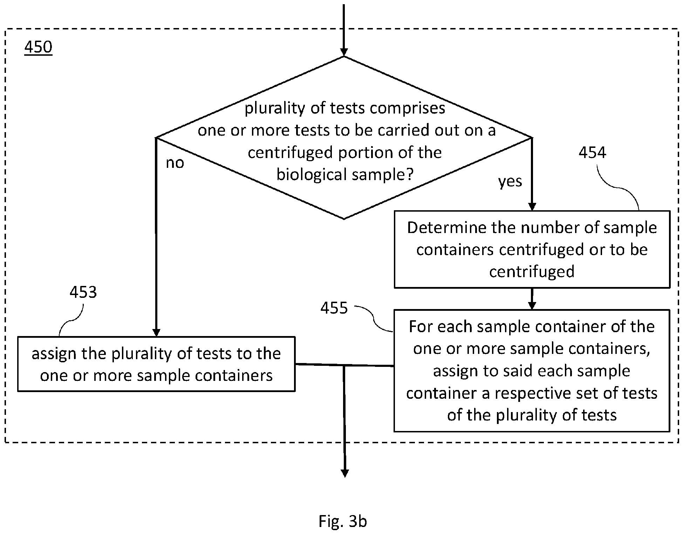

**Fig. 3b** show a second exemplary flowchart of step 450, schematically depicted in fig. 2b. Fig. 3b concerns in particular the case where aliquotation (i.e. preparation of one or more second sample containers) is carried out before step 450 so that, at the time the set of tests are assigned to the sample containers, a portion of the biological sample is contained in the first sample container and other portions of the biological sample are container in one or more second sample containers. In particular, in this case, the number of the plurality of sample containers is known.

In case it has been determined in step 440 that none of the tests is to be carried out on a centrifuged portion (i.e. all tests are to be carried out on an uncentrifuged portion), the computing device 100 carries out step 453, which is described above and schematically depicted in fig. 3a.

In case it has been determined in step 440 that at least one test is to be carried out on a centrifuged portion, steps 454 and 455 are carried out.

At step 454, the computing device determines the number of sample containers which are already centrifuged or which are to be centrifuged. Step 454 allows for determining also the number of sample containers which are uncentrifuged and not to be centrifuged, as the number of the plurality of sample containers is known. In particular step 454 comprises determining the number of sample containers which are uncentrifuged and are not to centrifuged.

At step 455, for each sample container of the one or more sample containers, the computing device 100 assigns to said each sample container a respective set of tests of the plurality of tests. At step 455, the assignment is based on the number of of sample containers which are uncentrifuged and are not to be centrifuged and the number of sample containers which are already centrifuged or which are to be centrifuged.

In particular, the centrifuged tests are assigned to the sample containers which are centrifuged or to be centrifuged so that those tests distributed evenly among those containers. For example, seven centrifuged tests are assigned to four sample containers, C1, C2, C3 and C4 which are centrifuged or to be centrifuged so that each of the sample containers C1, C2, C3 has two tests assigned thereto and C4 has one test assigned thereto. Also the uncentrifuged tests are assigned to the sample containers which are uncentrifuged and are not to be centrifuged so that those tests are distributed evenly among those containers. For example, ten centrifuged tests are assigned to four sample containers, C5, C6, C7 and C8 which are not centrifuged and not to be centrifuged so that each of the sample containers C5 and C6 has three tests assigned thereto and each of the sample containers C7 and C8 has two tests assigned thereto.

With reference to fig. 2a, the flowchart 401 may comprise optional step 470. At optional step 470, the sample containers that shall be centrifuged may be caused to be centrifuged. In particular, the sample containers that shall be centrifuged are the sample containers of the plurality of sample containers, to which centrifuged tests are assigned. More particularly, the computing device 100 determines, among the plurality of sample containers, the sample containers to which centrifuged tests are assigned.

For example, step 470 may comprise instructing, by the computing device 100, the ALS 200 to centrifuge the sample containers that shall be centrifuged, thereby initiating the centrifugation. For example, the computing device 100 may provide to the ALS 200 instructions specifying that the ALS 200 is to centrifuge the sample containers that shall be centrifuged. In particular, step 450 comprises centrifuging, by a centrifuge of the ALS 200, the sample containers that shall be centrifuged. For example, based on the instructions received by the computing device 100, the ALS 200 may instruct one of its centrifuges to centrifuge the sample containers that shall be centrifuged. The flowchart 401 may also comprise optional step 460, as described above and schematically shown in fig. 2a.

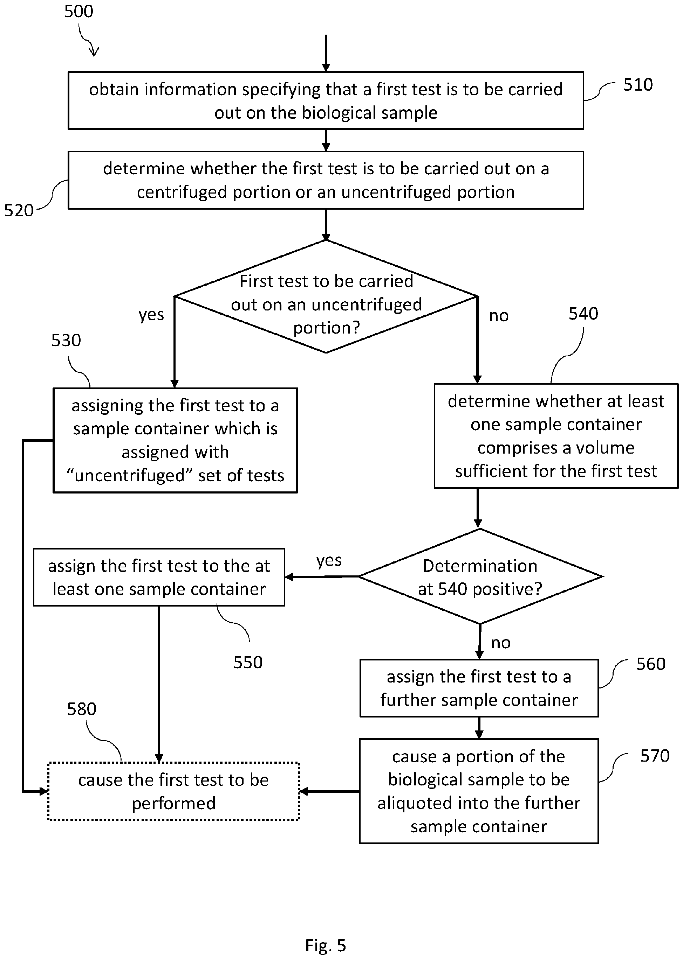

**Fig. 5** shows a flowchart 500 of an exemplary method which may be carried out after the method of fig. 2a or after the method of fig. 2b.

At step 510, the computing device 100 obtains information specifying that a first test is to be carried out on the biological sample. The first test may be a rerun or a reflex test. Said information may be based on a result of a second test of the plurality of tests, e.g., a test which has been caused to be performed at step 460 of fig. 2a or fig. 2b. For instance, obtaining the information may comprise determining, based on the result of the second test, that the first test is to be carried on the sample. In this case, in particular, a rerun or a reflex test (i.e., the first test) is to be carried to confirm or dispute the result of a prior test (i.e., the second test).

The first and second tests may be the same test, i.e., the result of the second test triggers a rerun of said test. For example, the first test may be the same test type of the second test, but it is to be carried out by a different laboratory analyzer. For instance, the first and second tests may be different from each other, i.e., the result of the second test triggers a reflex test, i.e., the first test.

At step 520, it is determined whether the first test is to be carried out on a centrifuged portion of the biological sample or is to be carried on a uncentrifuged portion of the biological sample.

If the first test is to be carried out on a uncentrifuged portion of the biological sample, the first test is assigned to a sample container or the plurality of sample container, such that the respective set of tests assigned to said sample container comprises only tests that are to be carried out on a uncentrifuged portion of the biological sample (step 530).

If the first test is to be carried out on a centrifuged portion of the biological sample, the computing device 100 determines whether at least one sample container of among the first subset of sample containers comprise a volume of biological sample that is sufficient for carrying out the first test (step 540). In the positive, the computing device 100 assigns the first test to the at least one sample container (step 550). Accordingly, the first test may be assigned to a sample container that is or is to be centrifuged, which streamlines the processing of the plurality of sample containers, as the performance of the first step does not require the centrifugation of the sample container to which the first test is assigned.

If no sample container of the first subset of sample containers comprises a volume of biological sample that is sufficient for carrying out the first test, the computing device 100 assigns the first test to a further sample container 560 and causes a portion of the biological sample to be aliquoted from a sample container of the second subset of the plurality of sample containers 570.

At optional step 580, the first test, and optionally other tests assigned to the sample container, is caused to be performed. For instance, step 540 comprises instructing, by the computing device 100, the ALS 200 to perform the first test, thereby initiating the performance of said test. For example, the computing device may provide to the ALS 200 instructions specifying that the ALS 200 shall perform the first test. In particular, step 580 comprises performing the first test, e.g., in response to the instructions provided by the computing device 100 to the ALS 200.

Throughout the description, including the claims, the term "comprising a" should be understood as being synonymous with "comprising at least one" unless otherwise stated. In addition, any range set forth in the description, including the claims should be understood as including its end value(s) unless otherwise stated. Specific values for described elements should be understood to be within accepted manufacturing or industry tolerances known to one of skill in the art, and any use of the terms "substantially" and/or "approximately" and/or "generally" should be understood to mean falling within such accepted tolerances.

Although the present disclosure herein has been described with reference to particular examples, it is to be understood that these examples are merely illustrative of the principles and applications of the present disclosure.

It is intended that the specification and examples be considered as exemplary only, with a true scope of the disclosure being indicated by the following claims.

A reference herein to a patent document or any other matter identified as prior art, is not to be taken as an admission that the document or other matter was known or that the information it contains was part of the common general knowledge as at the priority date of any of the claims.

## Claims

1. A method comprising:
• obtaining, by a computing device, a plurality of tests to be performed by an automated laboratory system on a biological sample,
• determining, by the computing device among the plurality of tests, one or more tests that are to be carried out on a centrifuged portion of the biological sample and one or more tests that are to be carried on a uncentrifuged portion of the biological sample; and
• for each sample container of the plurality of sample containers, assigning, by the computing device, a respective set of tests of the plurality of tests to said each sample container, such that said respective set of tests assigned to said each sample container comprises either only tests that are to be carried out on a centrifuged portion of the biological sample or only tests that are to be carried out on an uncentrifuged portion of the biological sample wherein each sample container of the plurality of container contains or is to contain a respective portion of the biological sample.

2. The method of claim 1, wherein, for each sample container of the plurality of sample containers, assigning the respective set of tests of the plurality of tests to said each sample container is such that:
- if the respective portion of the biological sample contained in said each sample container is centrifuged or is to be centrifuged, the respective set of tests comprises only tests that are to be carried out on a centrifuged portion of the biological sample, and
- if the respective portion of the biological sample contained in said each sample container is uncentrifuged and is not to be centrifuged, the respective set of tests comprises only tests that are to be carried out on an uncentrifuged portion of the biological sample.

3. The method according to any one of the preceding claims, wherein the respective portion of the biological sample contained or to be contained in at least one second sample container of the plurality of sample containers is respectively aliquoted or to be aliquoted from a first sample container of the plurality of sample containers, wherein
the method optionally further comprises, for the at least second sample container, causing the respective portion of the biological sample to be aliquoted from the first sample container into the at least one second sample container.

4. The method according to claim 3, wherein
the respective set of tests of the plurality of tests assigned to the first sample container comprises only tests that are to be carried out on an uncentrifuged portion of the biological sample.

5. The method according to any one of the preceding claims, further comprising:
• causing at least one third sample container of the plurality of sample containers to be centrifuged, wherein the respective set of tests of the plurality of tests assigned to the third sample container comprises only tests that are to be carried out on a centrifuged portion of the biological sample.

6. The method according to the preceding claim, wherein causing the third sample container to be centrifuged comprises one or more of: causing the third sample container to be capped with a removable cap before being centrifuged and causing the third sample container to be uncapped after being centrifuged.

7. The method according to any one of the preceding claims, wherein the respective set of tests assigned to a fourth sample container comprises only tests that are to be carried out on a centrifuged portion of the biological sample and the respective set of tests assigned to a fifth sample container comprises only tests that are to be carried out on an uncentrifuged portion of the biological sample.

8. The method according to any one of the preceding claims, further comprising: causing at least one test of the respective set of tests assigned to a sixth sample container of the plurality of sample container to be performed on a volume of the respective portion of the biological sample contained or to be contained in the sixth sample container.

9. The method according to any one of the preceding claims, further comprising:
obtaining information specifying that a first test is to be carried out on the biological sample, wherein said information is based on a result of a second test of the plurality of tests,
determining whether the first test is to be carried out on a centrifuged portion of the biological sample or is to be carried on a uncentrifuged portion of the biological sample;
if the first test is to be carried out on a uncentrifuged portion of the biological sample, assigning the first test to a sample container or the plurality of sample container, such that the respective set of tests assigned to said sample container comprises only tests that are to be carried out on a uncentrifuged portion of the biological sample.

10. The method according to the preceding claim, further comprising, if the first test is to be carried out on a centrifuged portion of the biological sample, assigning the first test to a sample container of the plurality of sample containers, such that the respective set of tests assigned to said sample container comprises only tests that are to be carried out on a centrifuged portion of the biological sample.

11. The method according to claim 9, further comprising, if the first test is to be carried out on a centrifuged portion of the biological sample:
• determining whether at least one sample container among one or more sample containers comprise a volume of biological sample that is sufficient for carrying out the first test, wherein the one or more sample containers are comprised in the plurality of sample containers and such that the respective sets of tests assigned to said one or more sample containers comprise only tests that are to be carried out on a centrifuged portion of the biological sample;
if at least one sample container among one or more sample containers comprises a volume of biological sample that is sufficient for carrying out the first test:
• assigning the first test to the at least one sample container; and if no sample container among the one or more sample containers comprises a volume of biological sample that is sufficient for carrying out the first test:
• assigning the first test to a further sample container; and
• causing a portion of the biological sample to be aliquoted from a seventh sample container of the plurality of sample containers into the further sample container, wherein the respective set of tests assigned to the seventh sample container comprises only tests that are to be carried out on an uncentrifuged portion of the biological sample.

12. The method according to any one of the preceding claims, further comprising, for each sample container of the plurality of containers, causing, by the computing device, the respective set of tests to be carried out on at least a volume of the respective portion of the biological sample, wherein optionally,
for each sample container of the plurality of containers, causing the respective set of tests to be carried out on the respective portion of the biological sample comprises instructing an automated laboratory system to carry out the respective set of tests on the respective portion of the biological sample.

13. A computing device comprising a processor configured to carry out the method according to any one of the preceding claims.

14. A computer program product comprising instructions which, when the program is executed by a computer system, cause the computer system to carry out the method according to any one of the method claims.

15. A computer-readable medium comprising instructions which, when executed by a computer system, cause the computer system to carry out the method according to any one of the method claims.
